# EUROPEAN PATENT APPLICATION

(11) **EP 1 470 806 A1**
(43) Date of publication of application: **27.10.2004**
(21) Application number: 02788828.8
(22) Date of filing: 13.12.2002
(51) Int. Cl.: A61F 5/44, A61F 5/453, A61F 5/455

(54) **ABSORPTIVE PRODUCT AND METHOD OF PRODUCING THE SAME AND METHOD OF HANDLING THE SAME, AND INNER BAG USED THEREFOR AND METHOD OF PRODUCING THE SAME AND FOOTGEAR USING THE SAME**

(30) Priority: 14.12.2001 JP 2001382171
(71) Applicant: Japan Absorbent Technology Institute, Chuo-ku, Tokyo 103-0007 (JP)
(72) Inventor: SUZUKI, Migaku Japan Absorbent Technology Inst., Chuo-ku, Tokyo 103-0007 (JP); MORIYA, Reiko Japan Absorbent Technology Inst., Chuo-ku, Tokyo 103-0007 (JP); SUGIYAMA, Katsuhiko Oji Paper Co. Ltd., Kasugai-shi, Aichi 486-0834 (JP); TAKEMATSU, Satomi Oji Paper Co. Ltd., Chuo-ku, Tokyo 104-0061 (JP)
(74) Representative: Gervasi, Gemma, Dr.
(86) International application number: PCT/JP2002/013078
(87) International publication number: WO 2003/051243

(57) **Abstract**

An absorptive product comprising (1) an individual-packaging sheet (11), (2) an absorptive body (12) placed on this individual-packaging sheet (11), (3) a trap sheet (14) forming, together with the individual-packaging sheet (11), a flexible trap portion (13) in which at least one end of the absorptive body (12) is received, and (4) a joining tape (15) installed on the trap sheet (14) and/or at the other end of the individual-packaging sheet (11) for releasably joining the other end of the individual-packaging sheet (11) and one end of the individual-packaging sheet (11) or trap sheet (14) in such a manner that the other end side of the individual-packaging sheet(11) overlaps the trap sheet (14).

## Description

### Technical Field

The present invention relates to a practical absorptive product of individual-packaging form that curbs the generation of garbage to the utmost, the method of producing the same, the method of handling the same and the underpants using the same.

### Background Art

As for the ordinary products being manufactured, and provided to the consumers after the distribution phase, the packaging form with a wrapping that enables each of the said products to be handled as an independent article is called the "individual-packaging". The commonplace individually packaged products in the area of the absorptive products are for women, such as the sanitary napkins, the inner bags for panties, and the light-case continence pads. Aside from the above, the disposable panties for traveling are also becoming popularized.

The following can be listed as the function commonly required for these individually packaged absorptive products:
(1) They are compact and thus can be carried about in a pochette and a handbag.
(2) A place or containers for disposal are provided, and the size of these products does not grow so big after use.
(3) They can be kept out of sight when being carried about, when in use or when disposed after use.
(4) They can be kept clean and therefore hygienic.

For example, in the case of the individually packaged sanitary napkins, to make it more convenient to take the product out of the individual-packaging pouch, the so-called one-way-wrap or one-step-wrap, with which the entire sanitary napkin can be extremely easily taken out of the individual-packaging pouch by simply pulling the release tape bonded to the individual-packaging pouch, is becoming mainstream. When an individually packaged sanitary napkin like these is used, the sanitary napkin is taken out of the individual-packaging pouch, and placed in place on the underpants. The individual-packaging pouch may be discarded then, or it may be used to wrap the used napkin before wrapping it further with tissue paper and throwing it into the sanitary bin.

In the case of the conventional absorptive product of individual-packaging with improved convenience, especially in the case of the sanitary napkin, because it is necessary to detachably retain united the individual-packaging pouch and the absorptive product with a peel-off sheet made of material such as silicon-coated paper and specially-treated film, there is a problem of expensive cost for individual-packaging and the constant generation of refuse, such as the individual-packaging pouches and peel-off sheets.

It is possible for the individual-packaging pouch, used for individually packaged sanitary napkins, to be discarded right after the sanitary napkin is taken out of it, resulting in the waste of resources, despite the fact that its individual-packaging pouch was originally developed for it to be used as a pouch in which to wrap the used napkin for disposal. Furthermore, if the individual-packaging pouch, after the sanitary napkin is taken out of it, is kept to be used as a pouch for wrapping and discarding the used napkin, it must be carried about in the pochette or handbag until the next replacement of the napkin and that is extremely bothersome.

At the same time, it is pointed out that applying the individually packaged absorptive product with the said (1) through (4) functions to the baby diapers and continence pads of large absorption capacity or adult urine pads, and further to adult diapers, is difficult in terms of cost justification and tractability at the time of disposal.

### Disclosure of Invention

An object of the present invention is to provide a practical absorptive product of individual-packaging form that curbs the generation of refuse to the utmost, the method of producing the same, the method of handling the same, and the inner bag used therefor, the method of producing the same and the underpants using the same, which are applicable to matters such as baby diapers, continence pads of large absorption capacity, adult urine pads, and adult diapers.

Preferred forms of the above-described invention are given below.
(1) The absorptive product of the present invention comprises an individual-packaging sheet, an absorptive body placed on this individual-packaging sheet, a trap sheet forming, together with the said individual-packaging sheet, a flexible trap portion in which at least one end of this absorptive body is held, and a joining tape attached on this trap sheet and/or at the other end of the said individual-packaging sheet to detachably join the other end of the said individual-packaging sheet and one end of this individual-packaging sheet or the said trap sheet, in such a manner that the other end of the said individual-packaging sheet overlaps the said trap sheet.
(2) The method of producing the absorptive product of the present invention is the method of producing the absorptive product comprising the trap sheet that has a penis-guiding section to guide the penis into the trap portion, the flexible liquid-impermeable inner bag that is incorporated in the trap portion and holds at least one end of the absorptive body, and the openings that are formed in the trap portion to allow parts of the folded portion of the inner bag to evaginate, and the method involves a step to form a pair of cross-directional straight-line crena along both side edges of the single sheet material to make an opening on each side of this single sheet material, a step to fold the said single sheet material in the width direction in such a manner to include the said pair of crena in the folded portion, and a step to form the trap portion by joining the folded portion that overlaps each other along the cross-sectional side edges of the said single sheet material.
(3) The method of handling the absorptive product of the present invention comprises a step to open the portion on the said other end of the said individual-packaging sheet by peeling off the said joining tape, a step to use the said absorptive product in this opened state, and a step, after use, to join, using the said joining tape, the said other end of the said individual-packaging sheet and one end of this individual-packaging sheet or the said trap sheet, by overlapping the said other end of the said individual-packaging sheet with the said trap sheet.
(4) The inner bag of the present invention is a flexible liquid-impermeable inner bag that is incorporated in the trap portion of the absorptive product of the present invention and holds at least one end of the absorptive body, and it has a folded portion to allow evagination, comprising the first pair of folded portion, one each on the right and left, formed by folding the right side and left side of the rectangular sheet toward the center, the second folded portion formed by folding the bottom end of the said sheet upward, the third folded portion formed by folding the top end of this second folded portion downward, and the hanger tape each of whose both ends is joined to the center of this third folded portion and the center of the top part of the said sheet.
(5) The method of producing the inner bag of the present invention consists of a step to form the first folded portion by folding both the right and left sides of the rectangular sheet toward the center, a step to form the second folded portion by folding the bottom end of the said sheet upward, a step to form the third folded portion by folding once again the top portion of the said second folded portion downward, and a step to join each of the both ends of the hanger tape to the center of the said third folded portion and the center of the top part of the said sheet.
(6) The underpants of the present invention have a part to which the absorptive product of the present invention or the absorptive product produced by the method of the present invention is fit, in such a manner that it is detachable.
(7) The underpants of the present invention comprise the absorptive product of the present invention or the absorptive product produced by the method of the present invention, and the part to which this absorptive product is fit, in such a manner that it is detachable.

In accordance with the absorptive product of the present invention, because it comprises an individual-packaging sheet, an absorptive body placed on this individual-packaging sheet, a trap sheet forming, together with the individual-packaging sheet, a flexible trap portion in which at least one end of this absorptive body is held, and a joining tape attached on this trap sheet and/or at the other end of the individual-packaging sheet to detachably join the other end of the individual-packaging sheet and one end of this individual-packaging sheet or the trap sheet, in such a manner that the other end of the individual-packaging sheet overlaps the trap sheet, by joining, after use, with the joining tape, the other end of the individual-packaging sheet and one end of this individual-packaging sheet or the said trap sheet, having the other end of the individual-packaging sheet and the trap sheet overlapping with each other, disposal of the used absorptive product while maintaining its sealed state is possible. Also, in case the absorptive body cannot absorb a great amount of liquid instantaneously, due to the liquid-impermeability of the trap portion, it is possible to temporarily hold the liquid within the trap portion, and eventually make the absorptive body retain a great amount of liquid.

Where at least the portion of the individual-packaging sheet, with which the absorptive body is overlapped, has a liquid-impermeable property, problems such as oozing out through the individual-packaging sheet of the liquid retained in the absorptive body can be prevented.

Where the absorptive body has a folded portion extending in a width-wise direction within the trap portion, because it increases the planar dimension of the absorptive body present within the trap portion, even in case a great amount of liquid is discharged into the trap portion in a short time, overflowing of the liquid to outside this trap portion can be controlled.

Where the absorptive body has a second folded portion extending in a longitudinal direction on each cross-directional side edge, it is possible to further increase the volume of liquid absorbed and retained by the absorptive body.

Where a dislocation prevention member is further provided between the individual-packaging sheet and the absorptive body, to prevent the absorptive body from dislocating against the individual-packaging sheet, it is possible to prevent the absorptive body from dislocating against the individual-packaging sheet.

Where the trap sheet has a penis-guiding section to guide the penis into the trap portion, it will make the absorptive product suitable for a men's continence pad.

Where a flexible liquid-impermeable inner bag is provided that is incorporated in the trap portion and holds at least one end of the absorptive body, it will be possible to give liquid-permeability to the individual-packaging sheet and the trap sheet and a comfortable fit will be obtained.

Where the inner bag has a folded portion whose outer margin, in the folded state, is held in the trap portion, and especially in case where the openings are further provided to allow parts of the folded portion of the inner bag to evaginate, even if the absorptive body cannot absorb all of a great amount of liquid and thus it is temporarily accumulated inside the inner bag, the inner bag's folded portion will expand and evaginate out of the trap portion through the openings, and thus such problems as an overflow of the liquid from the inner bag can be prevented.

Where the individual-packaging sheet and the trap sheet are made of the same sheet material, these can be easily produced with a single sheet of material.

Where the other end of the trap sheet has liquid-permeability, especially if it further has a guiding section, joined to the portion of trap sheet where it has liquid-permeability, facing the female wearer's urinary meatus to guide the urine to the absorptive body in a dispersed state, it will make the absorptive product suited for a women's continence pad.

Where a pair of elastic members are further provided along the right and left side edges of the individual-packaging sheet, joined in the stretched state to both the right and left side edges of the individual-packaging sheet, when the absorptive product is in use, its close fit to the skin can be improved.

Where the absorptive body has a non-woven substrate in a sheet form and there are multiple liquid-absorbing portions coated on this non-woven substrate at prescribed intervals, especially when the absorptive body contains 60 weight % or more of SAP, the volume can be made compact, to less than 1/3 compared to such conventional products as thin-type diapers, making possible the provision of the various types of absorptive products for children and adults.

Where the absorptive body has a hydrodisintegrative characteristic, disposal as it is, without any kinds of special treatment, will be possible.

Where the individual-packaging sheet makes up the absorptive product's liquid impermeable barrier material, the leak prevention function and other special individual packaging materials for the absorptive body can be omitted, and furthermore, it becomes possible for an inexpensive film material to make up the individual-packaging sheet, and thus its production cost can be greatly reduced. Furthermore, no individual-packaging materials will have to be prepared, making it possible to reduce the amount of garbage to be disposed.

In accordance with the method of producing the absorptive product of the present invention, the absorptive product of the present invention can be easily and rationally produced because a pair of cross-directional straight-line crena are formed along both side edges of the single sheet material, to make an opening on each side of this single sheet material, and the single sheet material is folded in the width-wise direction in such a manner as to include the pair of crena in the folded portion, to form the trap portion by joining the folded portion that overlaps each other along the cross-sectional side edges of the single sheet material.

In accordance with the method of handling the absorptive product of the present invention, because the portion on the other end of the individual-packaging sheet is opened by peeling off the joining tape, the absorptive product is used in this opened state, and, after use, the other end of the individual-packaging sheet and one end of this individual-packaging sheet or the trap sheet, by overlapping the other end of the individual-packaging sheet with the trap sheet, are joined with the joining tape, especially in the case where it is discarded after use in the state as the other end of the individual-packaging sheet and one end of this individual-packaging sheet or the trap sheet are joined, the sealed state of the used absorptive product can be maintained, thus the disposal in the preferable hygienic condition for the user as well as for the environment becomes possible, resulting in making it an absorptive product with an extremely high level of convenience.

In accordance with the inner bag of the present invention, it is possible to ensure the formation of the portions that are allowed to evaginate, without making a complicated accordion structure, but by simply folding the rectangular film and by joining with the hanger tape at the predetermined locations.

In accordance with the method of producing the inner bag of the present invention, it is possible to produce the inner bag extremely easily and at low cost, by simply folding the rectangular film and by joining with the hanger tape at the predetermined locations.

In accordance with the underpants of the present invention, putting on and taking off of the absorptive product to and from the underpants can be easily done, because there is a part to which the absorptive product of the present invention is fit, in such a manner that it is detachable.

In accordance with the underpants of the present invention, it is possible to remove the used absorptive product from the underpants and fit the new absorptive product to the underpants, because there are the absorptive product of the present invention, or the absorptive product produced by the method of the present invention, and the part to which this absorptive product is fit, in such a manner that it is detachable.

Where the part to fit is provided inside the underpants, it is possible to prevent such problems as the absorptive product's being dislocated from the underpants' prescribed location while in use.

Where the joining tape of the absorptive product is further equipped with joining portions that enable detachable joining, it is possible to more reliably prevent such problems as the absorptive product's being dislocated from the underpants' prescribed location while in use.

Where the joining portion is provided outside the underpants, because the joining portion does not touch the wearer's skin directly, a comfortable fit is obtained.

Where the underpants are of the plain knit product, a close fit of the absorptive product to the wearer's skin can be ensured.

### Brief Description of Drawings

Fig. 1 is an oblique perspective figure of the external appearance of an example of the absorptive product in accordance with the present invention, illustrating either the unused state or the disposal state.
Fig. 2 is a longitudinal section view of an example shown in Fig. 1.
Fig. 3 is a plan view of an example shown in Fig. 1 in its opened state.
Fig. 4 is an oblique perspective figure of the external appearance of an example of the absorptive body in the present invention.
Fig. 5 is an oblique perspective figure of the external appearance of a different example of the absorptive body in the present invention.
Fig. 6 is a plan view of the external appearance of yet another different example of the absorptive product in accordance with the present invention, illustrating either the unused state or the disposal state.
Fig. 7 is a plan view of another example of the absorptive product in accordance with the present invention in its opened state, illustrating the state where the absorptive body is excluded.
Fig. 8 is a plan view of yet another example of the absorptive product in accordance with the present invention in its opened state, illustrating the state where the absorptive body is excluded.
Fig. 9 is a plan view of the external appearance of an example where the absorptive product in accordance with the present invention is applied to the women's continence pad.
Fig. 10 is an oblique perspective figure of the external appearance of an example of the disposal state of the absorptive product in accordance with the present invention.
Fig. 11 is a plan view of the external appearance of another example where the absorptive product in accordance with the present invention is applied to the women's continence pad.
Fig. 12 is a plan view of the external appearance of an example where the absorptive product in accordance with the present invention is applied to the men's continence pad.
Fig. 13 is a plan view of the external appearance of another example where the absorptive product in accordance with the present invention is applied to the men's continence pad.
Fig. 14 is a plan view of the external appearance of a different example where the absorptive product in accordance with the present invention is applied to the men's continence pad.
Fig. 15 is a plan view of the external appearance of another different example where the absorptive product in accordance with the present invention is applied to the men's continence pad.
Fig. 16 is a sectional view of the example shown in Fig. 15, taken along the line XVI to XVI.
Fig. 17 is an oblique perspective figure of the external appearance of the inner bag in the example shown in Fig. 15.
Fig. 18 is an illustrative oblique perspective figure of the external appearance of the inner bag shown in Fig. 17 in the state where the liquid is temporarily held in it.
Fig. 19 is an oblique perspective figure of the external appearance of the absorptive body in the example shown in Fig. 15.
Fig. 20 is a plan view of the individual-packaging sheet and the trap sheet in the example shown in Fig. 15 in the opened state,
Fig. 21 is a conceptual rendering of the example shown in Fig. 15 in use.
Fig. 22 is an inside view of an example where the underpants in accordance with the present invention are applied to the men's brief.
Fig. 23 is a side view of an example of the portion of the joining tape in the present invention.
Fig. 24 is an inside view of an example involving the underpants in accordance with the present invention.
Fig. 25 is an inside view of an example involving the underpants in accordance with the present invention.
Fig. 26 is an inside view of an example involving the underpants in accordance with the present invention.
Fig. 27 is an inside view of an example involving the underpants in accordance with the present invention.
Fig. 28 is an inside view of an example involving the underpants in accordance with the present invention.
Fig. 29 is an inside view of an example involving the underpants in accordance with the present invention.
Fig. 30 is an inside view of an example involving the underpants in accordance with the present invention.

### Best Mode for Carrying Out the Invention

In the absorptive product of the present invention, the portion of the individual-packaging sheet where the absorptive body is overlapped may have a liquid-impermeable property.

The absorptive body may have either the folded portion within the trap portion extending in a width direction, or the second folded portion extending in a longitudinal direction on each of the cross-sectional side edges.

A dislocation prevention member may be further provided between the individual-packaging sheet and the absorptive body to prevent the absorptive body from dislocating against the individual-packaging sheet.

The trap sheet may have a penis-guiding section to guide the penis into the trap portion, and a flexible liquid-impermeable inner bag that is incorporated in the trap portion and holds the absorptive body may be further provided. This inner bag may have a folded portion whose outer margin, in the folded state, is held in the trap portion. In this case, openings may be provided to allow parts of the folded portion of the inner bag to evaginate. Furthermore, the individual-packaging sheet and the trap sheet may be made of the identical sheet material.

The other end of the trap sheet may have liquid-permeability. In this case, it may further have a guiding member, joined to the portion of trap sheet where it has liquid-permeability, facing the female wearer's urinary meatus to guide the urine to the absorptive body in a dispersed state.

A pair of elastic members may be further provided along the right and left side edges of the individual-packaging sheet, joined in the stretched state to both the right and left side edges of the individual-packaging sheet.

The absorptive body may have a non-woven substrate in a sheet form and there may be multiple liquid-absorbing portions coated on this non-woven substrate at prescribed intervals, and the absorptive body may contain 60 weight % or more of SAP.

The absorptive body may have a hydrodisintegrative characteristic.

The individual-packaging sheet may make up the absorptive product's individual-packaging material.

In accordance with the method of handling the absorptive product of the present invention, the step to discard after use in the state as the other end of the individual-packaging sheet and one end of this individual-packaging sheet or the trap sheet are joined may be further provided.

A part to fit may be provided inside the underpants of the present invention.

The joining tape of the absorptive product may be further equipped with the joining portions that enable detachable joining.

The joining portion may be provided outside the underpants.

The underpants may be a plain knit product.

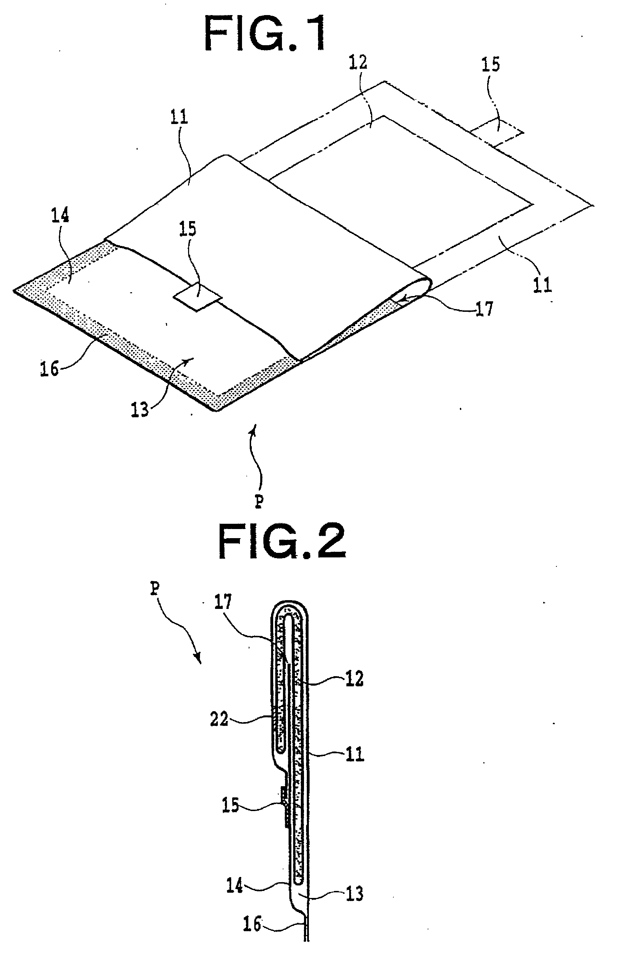

The examples of the absorptive product, the method of producing the same, and the method of handling the same, as well as the underpants using the same, in accordance with the present invention will be explained in further detail with reference to Fig. 1 through Fig. 30. The present invention, however, is not limited to these examples only, in that further combination of these examples as well as any and all changes and modifications included in the concept of the present invention described in the patent's claims are possible. The present invention, therefore, can be applied as a matter of course to other technologies which belong to the concept of the present invention.

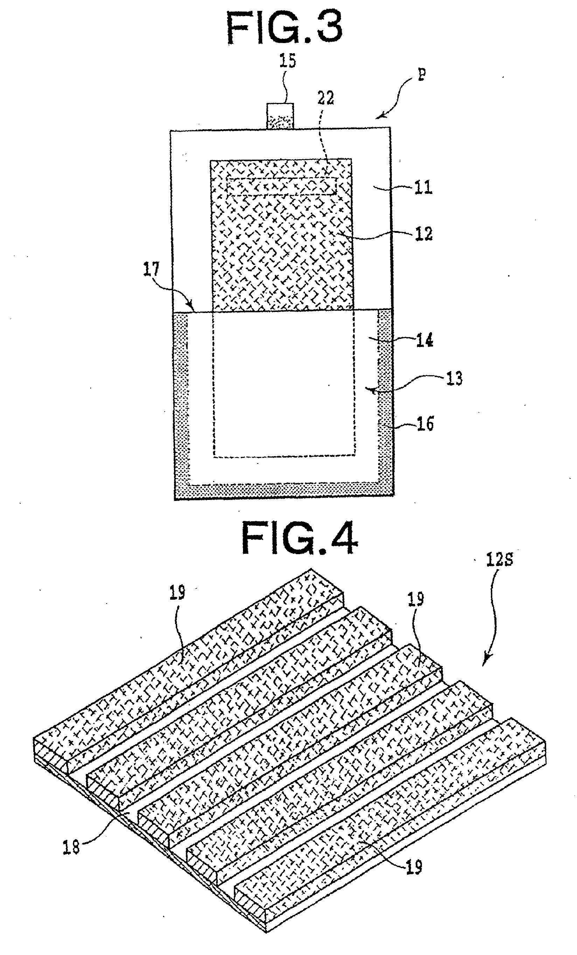

An external appearance of the first example is shown in Fig. 1, its vertical cross-sectional structure is shown in Fig. 2, and its opened state is shown in Fig. 3. That is, the absorptive product P in the present example comprises a liquid-impermeable individual-packaging sheet (11) that makes up the individual-packaging material, an absorptive body (12) integrally placed on this individual-packaging sheet (11), a trap sheet (14) forming, together with the individual-packaging sheet (11), a flexible trap portion (13) in which at least one end (generally goes on the lower side when in use) of the absorptive body (12) is held, and a joining tape (15) attached on the trap sheet (14) and/or at the other end of the individual-packaging sheet (11) to detachably join the other end of the individual-packaging sheet (11) and one end of the individual-packaging sheet (11) or trap sheet (14) in such a manner that the other end of the individual-packaging sheet(11) overlaps the trap sheet (14).

The trap portion (13) formed by the individual-packaging sheet (11) and the trap sheet (14) holds at least one end of the absorptive body (12), and depending on the type of use, the trap portion (13) may be in one case maintained in a sealed condition to protect itself from liquid leakage and in the other case may allow liquid permeation by concomitantly using the liquid-impermeable inner bag which will be discussed later.

As a type of use for which the trap portion (13) needs to be kept in the sealed state, in other words, in the liquid-impermeable state, the first example to be cited may be the case in which the absorptive body (12) to be held in the trap portion (13) is not covered with a heavy-duty retention sheet such as the top sheet and the back sheet, and the absorptive material such as SAP or pulp is only wrapped with thin covering material such as tissue paper. In this case, because it is necessary to prevent liquid leakage from the absorptive material, a liquid-impermeable characteristic is given to the trap portion (13). The second example is the case where it is used for the purpose of urine treatment especially targeted to men.

As a type of use for which the trap portion (13) needs to be kept in the sealed state, in other words, in the liquid-impermeable state, the first example to be cited may be the case in which the absorptive body (12) to be held in the trap portion (13) is not covered with a heavy-duty retention sheet such as the top sheet and the back sheet, and the absorptive material such as SAP or pulp is only wrapped with thin covering material such as tissue paper. In this case, because it is necessary to prevent liquid leakage from the absorptive material, a liquid-impermeable characteristic is given to the trap portion (13). The second example is the case where it is used with the purpose of urine treatment especially targeted to men. SAP is able to retain a large amount of liquid, but at the same time it has the characteristic of slowly absorbing the liquid, and when the absorptive body (12) that contains a high ratio of SAP with these characteristics is used, SAP's speed of absorbing urine cannot keep up with the speed of urinary drainage, thus requiring a measure to prevent the absorptive body (12) from leaking the urine which is more than it can absorb. In a case like this, the liquid-impermeable trap portion (13) can be made to function as a flexible container to temporarily hold urine.

As a type of use for which the trap portion (13), especially the trap sheet (14), is made to have liquid-permeability, the first example is the case where the liquid-impermeable inner bag is concomitantly used with it, and this inner bag is held in the trap portion (13). In this case, because the trap portion (13) has no other functions but to hold the inner bag, it is possible to use a highly-porous or mesh-type trap sheet (14), as well as to construct the trap sheet (14) with such materials as hydrophilic non-woven fabric. The second example is the case where it is used as a female urine pad. In this case also, at least one end (the bottom end) of the trap sheet (14) needs to be made liquid-impermeable to prevent the absorptive body (12) from leaking the urine which is more than it can absorb.

Both the individual-packaging sheet (11) and the trap sheet (14) of the present example are made of a liquid-impermeable and flexible PE sheet or a laminated sheet of PE film and non-woven fabric, and the trap portion (13) with an opening at the other end (top end) of the trap sheet (14) can be formed by overlapping the trap sheet (14) with the individual-packaging sheet (11) at one longitudinal end of the latter and making the heat-sealed portion (16) by heat-bonding the outer peripheral edges of the individual-packaging sheet (11) where the trap sheet (14) overlaps it.

Where a single synthetic film material is used for both the individual-packaging sheet (11) and the trap sheet (14), it is also possible to form the individual-packaging sheet (11) and the trap sheet (14) so that they have a trap portion (13), by folding a long sheet and heat-bonding both cross-sectional side edges of this long sheet's folded portion.

The other end of the absorptive body (12) of the present example is exposed through the opening (17) of the trap portion (13) and extended to the other end of the individual-packaging sheet (11). In the unused individually-packaged state shown in Fig. 1 and Fig. 2, the absorptive body is folded along with the individual-packaging sheet (11), at about the middle in the longitudinal direction of the absorptive sheet (11), for the other end of the absorptive body to be on top of the trap sheet (14) and to be in the state of being covered with the individual-packaging sheet (11). By making it return to the folded state shown in Fig. 1 and Fig.2 from the state in use shown in Fig. 3 that illustrates the unfolded state of this folded portion, disposal of the used absorptive product P without soiling the hands is possible.

By using a liquid-impermeable individual-packaging sheet (11), the absorptive body (12) can be constructed with nothing more than just the ordinary absorptive materials that primarily use such absorptive materials as SAP and wood pulp and a surface covering sheet such as a liquid permeable top sheet or tissue paper to be used to wrap them and to face the individual-packaging sheet (11). Simplification of the structure of the absorptive body (12) as found in this case not only allows simplification of the design of the absorptive product P and cost reduction, but also offers a great advantage in relation to user-friendliness in waste disposal.

For the compact construction of the absorptive product P, and further for the hygienic treatment of urine, it is preferable that the absorptive body (12) is extremely thin and has excellent morphological stability both before and after absorption, so that at least one end of it is smoothly and stably held inside the trap portion (13) and its shape is stably maintained after the swelling caused by liquid absorption. From this standpoint, the absorptive body (12) used in the present invention should have much a higher SAP content ratio than the SAP/pulp ratio seen in ordinary disposable diapers and sanitary napkins, and further, it is preferable that an absorptive body in a sheet-form that can stably maintain its shape, such as the one described in the Japan Patent Application Laid-open Hei 11-34200, is used.

Fig. 4 is an illustrative expression of the external appearance of such an absorptive body in a sheet-form (12S). This absorptive body in a sheet-form (12S) is constructed by making the highly absorbent areas (19), coated, by applying a line-coating technique like curtain-coating, in such a manner as to create multiple band-shapes in parallel with one another on the surface of the base sheet (18) along the longitudinal direction of the base sheet (18), made of non-woven fabric, preferably with bulkiness and air-permeability. In these band-shaped highly absorbent areas (19), 60% or more SAP, preferably 80 to 90%, is contained. The absorptive body in a sheet-form like this (12S) has excellent liquid-dispersing capability, due to the combination of the highly absorbent areas (19) and the areas without it where the base sheet (18) is exposed.

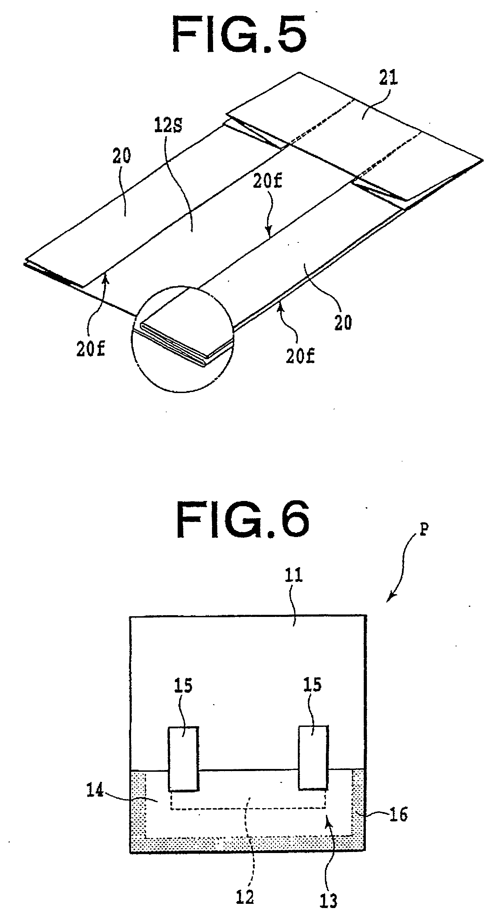

In order not to spoil expandability of the absorptive body in a sheet-form (12S), it is preferable that no surface-covering sheet such as non-woven fabric, tissue paper, or highly-porous film is used for this absorptive body in a sheet-form (12S) and that at least one end of it is held in the trap portion (13). As required, however, it can also be folded in a certain configuration and then a part of it can be inserted in the trap portion (13). For instance, as shown in Fig. 5, by forming the folded portions (20), one each along both cross-sectional side edges of the absorptive body in a sheet-form (12S), and further, the folded portion (21) by folding inwardly one end of this absorptive body in a sheet-form (12S), and then inserting this folded portion (21) in the trap portion (13) to be held there, absorption efficiency of the urine discharged in the trap portion (13) can be improved. A configuration such as this one, for instance, is suited for the absorptive body (12) to be used in men's continence pads, because it improves the ease of retention of the penis inserted between the two folded portions (20), on right and left. The folded portions (20) in the present example are in an accordion-form with two fold-lines (20f) on both sides in the longitudinal direction of the absorptive body in a sheet-form (12S).

By adopting a so-called flushable material, which can be flushed away in the toilet, for the base sheet (18), and by adopting a bio-degradable aminoacid SAP, it will be possible, as required, to remove the used absorptive body (12) from the trap portion (13) and then safely dispose of it by flushing it away in the toilet.

The anchoring end of the joining tape (15) is integrally joined to the center of the other end of the individual-packaging sheet (11), and the adhesive, not shown in the figures, is applied to the other end, which is detachably joined to the trap sheet (14). The adhesive is not applied on the very end edge of the joining tape (15) of the present example, for the sake of ease in peeling-off the joining tape (15), by pinching this end with the fingers. The functions of this joining tape (15) are, firstly to maintain the individual-packaging sheet (11) of the absorptive product P in the folded state as shown in Fig. 1 and to keep it in a hygienic and sealed state, and secondly to ensure ease in opening the folded individual-packaging sheet (11) and the other end of the absorptive body (12) at the time of use. Aside from these, it is also possible to let the joining tape (15) function to fix the location of the absorptive product P on the underpants, when the absorptive product P is fit into underpants such as panties and briefs.

In case a function of fixing the location of the absorptive product P on the underpants is given to the joining tape (15), increasing the width of the joining tape (15) or using multiple joining tapes (15) with some space in between, as shown in Fig. 6 (two joining tapes in this Fig.) will be effective in strengthening the joining force.

It is preferable that the joining tape (15) of the present invention has a stable re-joining capability, to enable re-joining, after the absorptive product P is used, of the individual-packaging sheet (11), folded as shown in Fig. 1 to the trap sheet with the joining tape (15), or joining of the joining tape (15), to the individual-packaging sheet (11) after it wraps the used product in such a manner as to roll in the trap portion together (13). From this standpoint, aside from the adhesive tape used in the present example, it is also possible to use as the joining tape (15) such hook and loop fasteners as Magic Tape (registered trademark) or Velcro (registered trademark). In this way, the absorptive product P can be disposed of not only without distorting the shape of the absorptive body (12), but also hygienically. At the time of disposal of this absorptive product P, because the absorptive body (12) is in a swelled state due to urine absorption, the packaging configuration after use will not be as compact as shown in Fig. 1, but basically there will be no major shape change. In response to the swelling of the absorptive body (12), by adopting as the joining tape (15) the multiple-fold type often attached to the pants-type diapers, re-joining by the joining tape (15) to the individual-packaging sheet (11) or the trap sheet (14) can be ensured.

In case the depth of the trap portion (13) is shallow in comparison to the length of the absorptive body (12), in order to prevent dislocation of the absorptive body (12) against the individual-packaging sheet (11), a dislocation-prevention member (22) is provided between the individual-packaging sheet (11) and the absorptive body (12). In the present example, a hot-melt adhesive is used as the dislocation-prevention member, but because the absorptive body (12) does not have to be permanently fixed to the individual-packaging sheet (11), it is also possible to allow the absorptive body to be removed from the individual-packaging sheet (11) by using the adhesive or the hook and loop fastener. Nothing more than just the prevention of dislocation by friction, using the anti-slip material as the dislocation-prevention member (22), also has some effect. The location, shape and the number of this dislocation-prevention member (22) to be used can be chosen randomly.

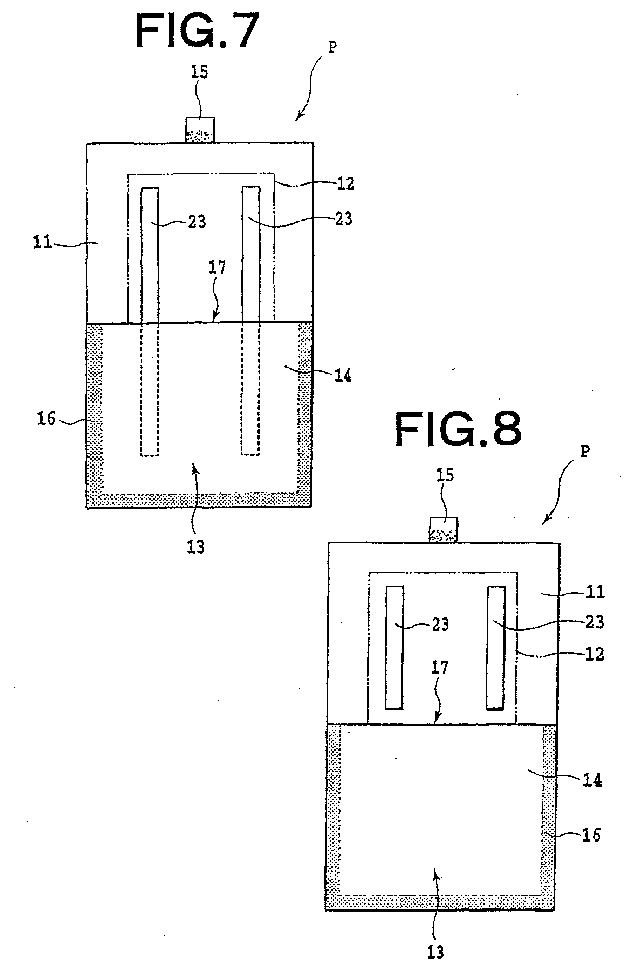

In order to bend the absorptive product P to fit the wearer's contour, it is also effective to deform the absorptive product P with the contractile force of the elastic member that is joined, in the stretched state by about, for instance, 20%, to both side edges in width-wise direction of the individual-packaging sheet (11). The other example of the absorptive product P in its opened state is shown in Fig. 7, in which the absorptive body (12) is omitted for the sake of convenience. The elastic member (23) is placed along both the width-wise side edges of the individual-packaging sheet (11), almost throughout its longitudinal length, but it is also acceptable to place the elastic member (23), in its stretched state, only on the other end of the individual-packaging sheet (11) where the trap sheet (14) is not overlapped, as shown in Fig. 8, illustrating yet another example in its opened state.

Where there is no external force applied to the absorptive product P, the elastic member (23) acts to make the width-wise side edges of the individual-packaging sheet (11) contract in a longitudinal direction against the other parts. Due to this, when it is put on the wearer's body, both of the width-wise side edges of the absorptive product P are deformed to closely fit the body, preventing the leakage of urine from the width-wise side edges.

The absorptive product P of the present invention has the above-mentioned examples as its basic components, and the desirable type of use can be obtained by adding or changing the functions, depending on the use. The absorptive product P of the present invention will be sequentially explained per limited use, but as for the functional elements identical to those of the examples already treated, they will only be marked with identical codes and the redundant explanations will be omitted.

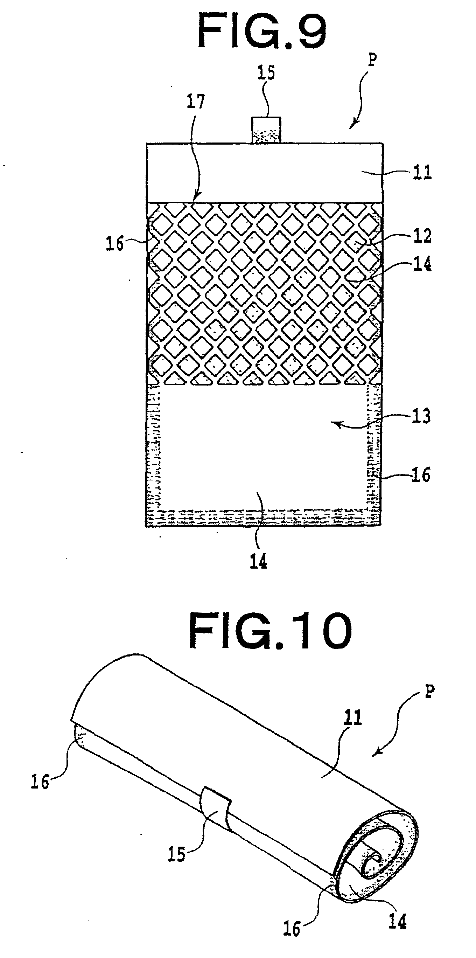

Fig. 9 through Fig.11 show examples of the application of the absorptive product P of the present invention to a female continence pad or urine pad, and all of them but Fig. 10 illustrate the examples in their opened state. Each of these examples has a structure that allows the absorptive body (12) to be put in and taken out by forming an opening (17) in the trap portion (13), but it is also possible to make it impossible to take out the absorptive body (12), by joining the outer margin of the trap sheet (14) entirely to the trap sheet (11). Fig. 10 shows an example of disposing the used absorptive product P by rolling it with its trap sheet (14) facing inside, but it is naturally also possible to dispose of it in a folded state, as seen in the other previous examples.

The example shown in Fig. 9, uses the liquid-permeable mesh sheet for the top half of the trap sheet (14) and the lower half is made liquid-impermeable, which makes it possible to treat a large amount of urine by temporarily guiding the amount of urine that is more than what the absorptive body (12) can absorb to this area.

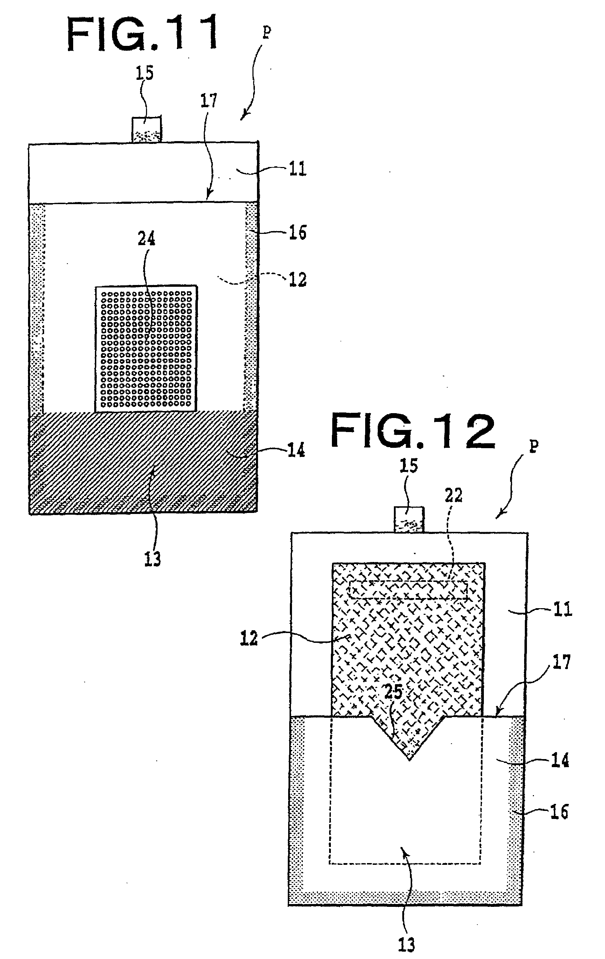

The example shown in Fig. 11 has the guiding member (24) joined to the center part of the liquid-permeable trap sheet (14) in a protruded state, in order to let it face the female urinary meatus and guide the urine to the absorptive body (12) in a dispersed state. The guiding member (24), of similar construction to sanitary tampons and plastic foam, has the function of quickly absorbing the discharged urine and guiding it to the absorptive body (12) via the trap sheet (14), making it possible to prevent leakage. In the present example also, one end of the trap sheet (14) (the shaded area in the figure) is kept liquid-impermeable to enable temporary holding of the urine in it.

Fig. 12 through 15 show examples of the application of the absorptive product P of the present invention to a male continence pad and urine pad.

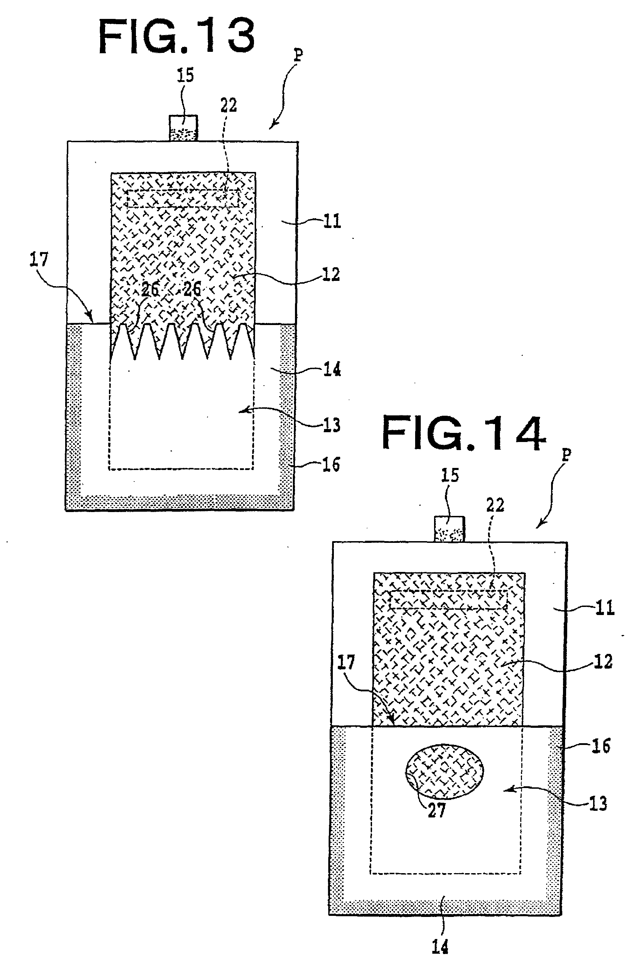

Fig. 12 through Fig. 15 show examples of the application of the absorptive product P of the present invention to the men's continence pad and urine pad. All of the examples shown in Fig. 12 through Fig. 16 have the penis-guiding section formed on the trap sheet (14) to guide the penis into the trap portion (13), while the penis-guiding section in the example shown in Fig. 12 is a crena in a V-canaliform (25) formed in the center of the top end of the trap sheet (14), the penis guiding section in the example shown in Fig. 13 is made up of a group of parallel crenas (26) in a multiple-gather configuration formed on the top end of the trap sheet (14), and the penis-guiding section in the example shown in Fig. 14 is a round opening (27) formed on the top end of the trap sheet (14). For the examples shown in Fig. 12 through Fig. 14, the liquid-impermeable trap sheet (14) must be adopted, but when the flexible liquid-impermeable inner bag is incorporated in the trap portion (13), the liquid-permeable material can be used as the trap sheet (14).

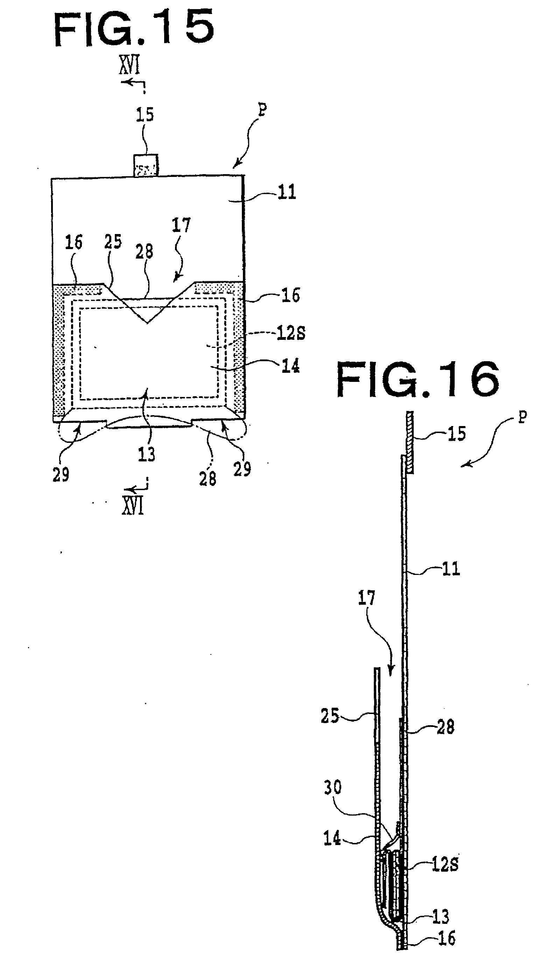

Fig. 15 and Fig. 16 that shows a sectional view of the example shown in Fig. 15, taken along the line XVI to XVI, show the example in which an inner bag (28) like this is incorporated and, in this example, the entire absorptive body (12) is held in the inner bag (28). On both left and right width-wise edges on the bottom end of the trap portion (13), a crena (29) on each edge is formed in such manner that it spreads over outside and inside the trap portion (13), and when the urine accumulates in the flexible inner bag (28), this inner bag (28) is designed to expand and portions of it to evaginate through the crenas (29), as shown in the chain double-dashed line in Fig. 15, thus preventing the urine from overflowing from the inner bag (28) to the trap portion (13).

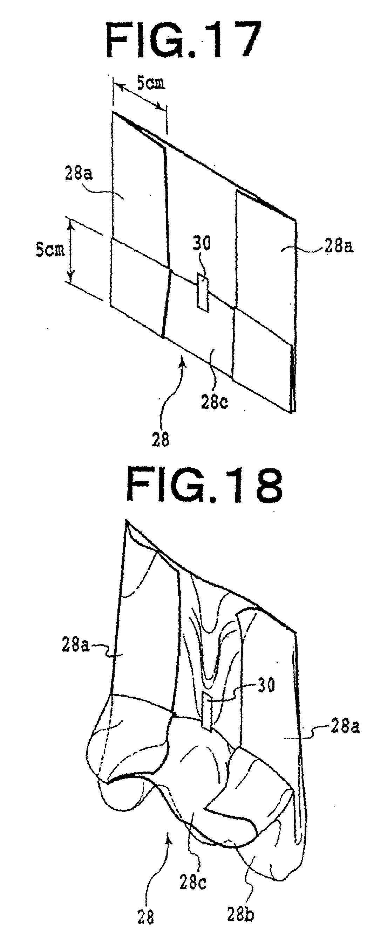

The external appearance of the inner bag (28) of this present example is shown in Fig. 17 and its external appearance in the state where the urine is temporarily accumulated is illustrated in Fig. 18. The inner bag (28) in the present example is made of Sanipak Company of Japan Ltd.'s PE film in a rectangular shape whose dimensions are, for instance, 28 cm in length, 24 cm in width and 0.02 mm in thickness, where the first folded portion (28a) is formed by folding inward the longer side by 5 cm each on both the left and right sides, and then the second folded portion (28b) is formed by folding upward the shorter side by 10 cm, and furthermore the third folded portion (28c) is formed by folding downward this second folded portion (28b). The center of the third folded portion (28c) and, located above it, the center of the top part on the shorter side end are joined on each end of the hanger tape (30). When urine is discharged into the inner bag (28), therefore, the inner bag (28) expands inside the trap portion (13) and its first and second folded portions (28a, 28b) located on each side of the hanger tape evaginate due to gravity, then these portions are designed to evaginate through each crena (29) formed in the trap portion (13). In this way, even if a large volume of urine is discharged, urine-leakage inside the trap portion (13) can be prevented.

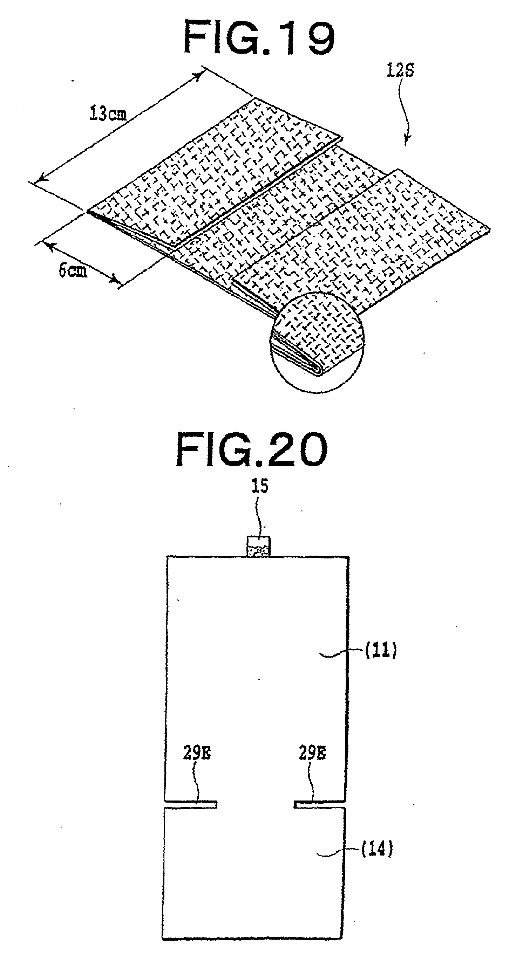

The external appearance of the absorptive body (12) of the present example, which is held in the inner bag (28), is shown in Fig. 19. That is, the absorptive body (12) of the present example is the absorptive body in sheet-form (12S), as shown in Fig. 4, whose trade name is MegaThin (Registered Trademark) made by Japan Absorbent Technology Institute, in a rectangular shape whose dimensions are, for instance, 28 cm in length and 26 cm in width, and with a folded construction formed by first folding its shorter side in half, and then by further folding inward on both left and right sides of it by 6cm.
The individual-packaging sheet (11) and the trap sheet (14) of this absorptive product P use, as shown in Fig. 20, the non-woven fabric (Type S.M.M.S.) made by Avgol, in a rectangular shape whose dimensions are, for instance, 38 cm in length, 20 cm in width and 20g/m² basis weight, and where the trap portion is formed by making a 7-cm-long crena (29E) each on both sides of the sheet at 14 cm from the longitudinal end of the sheet, then by folding the portion that makes the trap sheet (14) at these crenas (29E), and then by forming the heat-sealed portion (16) on both width-wise side edges as well as on right and left sides of the top edge of the folded trap sheet. In this case, prior to forming the heat-sealed portion (16) on the right and left sides of the top edge of the trap sheet (14), the inner bag (28) and the absorptive body (12) must be incorporated inside the trap portion (13). Although in the present example, the absorptive body (12), after it is folded, is held in its entirety inside the inner bag (28), the expandability of the absorptive body (12) can be maximized when only one end of it is held inside the inner bag (28), as shown in the previous examples.

While the above-mentioned absorptive product P can be used as it is by combing it with the conventional disposal diaper or underpants including shorts and pants, the dedicated underpants with a part that can hold this absorptive product P in the prescribed location are also a preferable mode for carrying out the present invention.

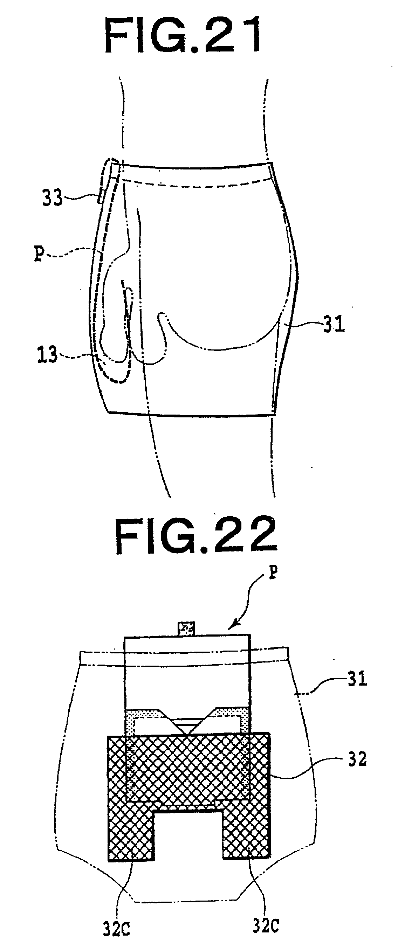

Fig. 21 through Fig. 30 show each of such examples of the underpants in accordance with the present invention, and it is preferable that all these underpants are made of plain knit material such as jersey knit to ensure excellent fit to the skin when worn.

Fig. 21 shows the urine pad also shown in Fig. 15 through Fig. 19, or the underpants (31), designed to suit the absorptive product P, in the state of being put on a wearer, and Fig. 22 is an illustrative drawing of its inner structure. The mesh pocket (32) with escapes (32C) to accommodate the portions of inner bag (28) that can evaginate through the crenas (29) is sewn on inside the underpants (31), as a part to put the product of the present invention. The external front body of these underpants (31) has a joining point (33) in Fig. 21 to detachably join the joining tape (15), which ensures stable retention of the absorptive product P inside the underpants (31) when worn, and thus prevent dislocation of it.

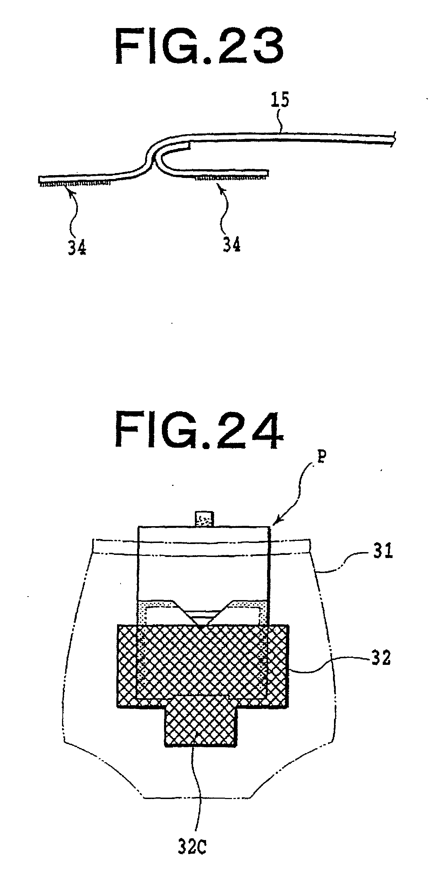

The enlarged view of the structure of the absorptive product P's joining tape (15) which is suitable for the joining point (33) on these underpants (31) is shown in Fig. 23. This joining tape (15) is of a multiple-fold-type whose pair of joining surfaces (34) can face each other and are connected at the anchoring end in such a manner that each of them can be turned over, and the location where the joining surfaces (34) are turned over can be freely altered, while either the back or the front surface can be used for joining. In the example shown in the figure, a hook and loop faster is placed on each of the joining surfaces (34), while the hook and loop fasters matching these fasteners are placed at the joining points (33) on the underpants (31).

The underpants (31) of the present example shown in Fig. 24 are made to match the absorptive product P and have a crena (29), the same as those of the urine pads shown in Fig. 15 through Fig.19, formed in the center of the bottom edge of the trap portion (13), and a mesh pocket (32) with an escape (32C) that is formed in the center of the bottom end and that can accommodate a portion of the inner bag, not shown in the figure, that evaginates through this crena, is sewn on the inside of the underpants (31).

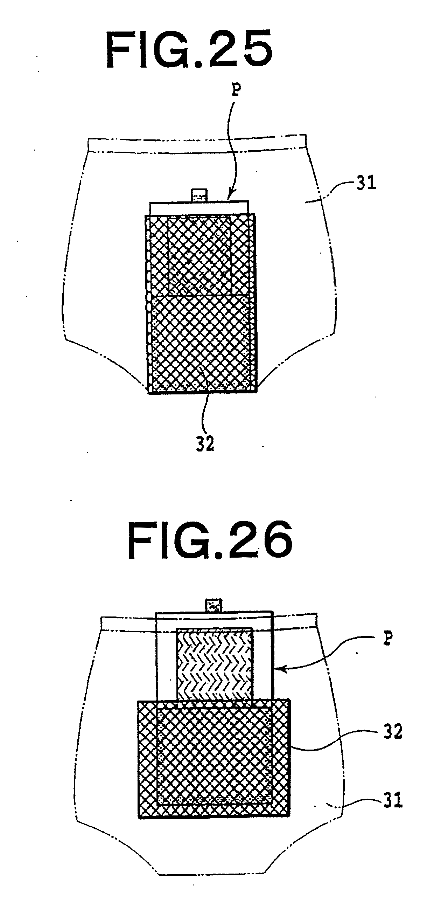

The underpants (31) in the example shown in Fig. 25 have a vertically long mesh pocket (32), that can hold the absorptive product P shown in Fig. 1 through Fig. 3, sewn on the inside of the underpants (31).

The underpants (31) in the example shown in Fig. 26 have a horizontally long mesh pocket (32), that can hold the absorptive product P, wider than the absorptive product P shown in Fig. 1 through Fig. 3, sewn on the inside of the underpants (31).

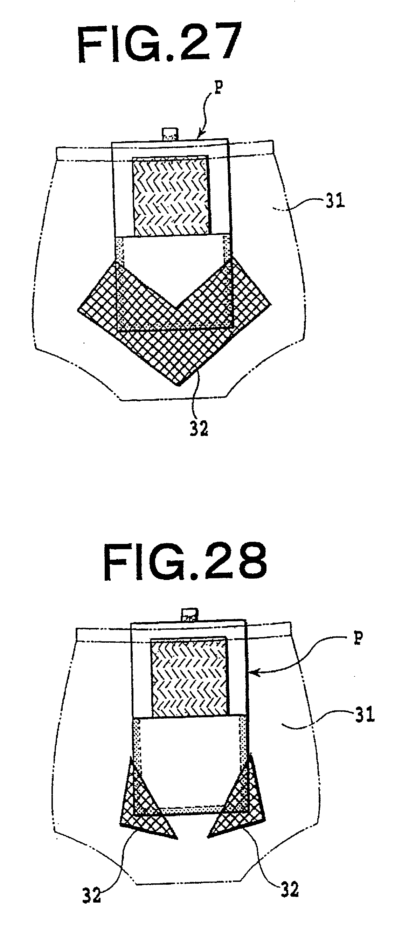

The underpants (31) in the example shown in Fig. 27 have a mesh pocket (32), that can hold the absorptive product P shown in Fig. 1 through Fig. 3, sewn on the inside of the underpants (31). It is in the shape of a letter V instead of rectangular but this sort of shape can also hold the absorptive product P.

The underpants (31) in the example shown in Fig. 28 have a mesh pocket (32), that can hold the absorptive product P shown in Fig. 1 through Fig. 3, sewn on the inside of the underpants (31), but it has a structure split in two to hold only the right and left ends of the absorptive product P.

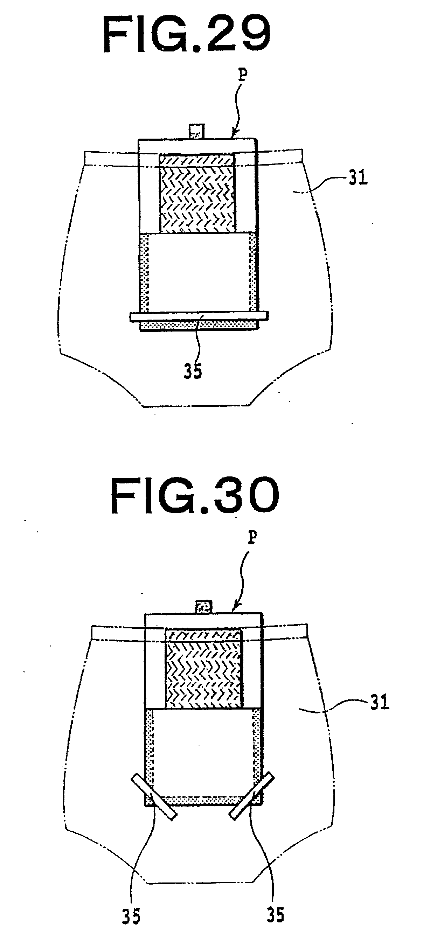

The underpants (31) in the example shown in Fig. 29 have a stretchable elastic member (35) in a ribbon shape, that can hold the absorptive product P shown in Fig. 1 through Fig. 3 by having the bottom end of it inserted between the underpants and the ribbon, while having only both ends of the ribbon being sewn on the inside of the underpants (31).

As shown in Fig. 30, it is also possible to attach a pair of elastic members (35) in an inclined state on the inside of the underpants (31), to hold both the right and left sides of the bottom end of the absorptive product P.

As described above, the structure and configuration of the part used to put the absorptive product on may be selected according to the type of use and other conditions. And at the same time, the joining points (33) placed on the front body outside the underpants (31) may also be changed depending on the configuration of the joining tape (15).

An experiment to verify the absorption effectiveness by actually putting on the absorptive product P (urine pad) shown in Fig. 15 through Fig. 19 was conducted. The basic characteristics of the absorptive body (12) used for the experiment are as follows:

| | |
|---|---|
| Basis Weight of the SAP | 150g/m² |
| Estimated Absorbed Volume | 55ml/g |
| Estimated Retention | 35ml/g |

The estimated absorbed volume of the entire absorptive body (12), therefore, is 400ml and the estimated retention 260ml.

The underpants (31) shown in Fig. 21, the L-size Cotton Trunks (Trade Name) made by Gunze Limited were put on a male test subject person, and then he was asked to urinate with the underpants on. 30 seconds after his urinating, the inner bag (28) was expanded to the maximum, and a portion of it evaginated through a crena (29), but no leakage of urine from the inner bag (28) to the trap portion (13) was observed. Next, 3 minutes after he started urinating, this absorptive product P was collected from the test subject person to measure the amount absorbed by the absorptive body (12). As a result, absorption of 430ml of urine was confirmed. This absorbed volume far exceeds the estimated retention of 260ml, as well as the estimated absorbed volume of 400ml. With this, it was verified that extremely effective absorption took place.

### Industrial Application

In accordance with the individually packaged absorptive product of the present invention and the underpants using the same, as well as inventions incidental to these, the generation of refuse can be curbed greatly. Because the absorptive product can maintain a sealed state after use, disposal in a hygienic manner, preferable for both the user and the environment, is possible. The absorptive product of the present invention, therefore, is extremely advantageous and has a great deal of potential in industry.

## Claims

1. The absorptive product, **characterized in that** it comprises an individual-packaging sheet, an absorptive body placed on this individual-packaging sheet, a trap sheet forming, together with the said individual-packaging sheet, a flexible trap portion in which at least one end of this absorptive body is held, and a joining tape attached on this trap sheet and/or at the other end of the said individual-packaging sheet to detachably join the other end of the said individual-packaging sheet and one end of this individual-packaging sheet or the said trap sheet, in such a manner that the other end of the said individual-packaging sheet overlaps the said trap sheet.

2. The absorptive product, according to claim 1, **characterized in that** at least the portion of the said individual-packaging sheet, with which the said absorptive body is overlapped, has a liquid-impermeable property.

3. The absorptive product, according to claim 1 or claim 2, **characterized in that** the said absorptive body has a folded portion extending in a width-wise direction within the said trap portion.

4. The absorptive product, according to any one of the claims 1 through 3, **characterized in that** the said absorptive body has a first folded portion extending in a longitudinal direction on each cross-directional side edge.

5. The absorptive product, according to any one of the claims 1 through 4, **characterized in that** a dislocation prevention member is further provided between the said individual-packaging sheet and the said absorptive body, to prevent the said absorptive body from dislocating against the said individual-packaging sheet.

6. The absorptive product, according to any one of the claims 1 through 5, **characterized in that** the said trap sheet has a penis-guiding section to guide the penis into the said trap portion.

7. The absorptive product, according to any one of the claims 1 through 6, **characterized in that** a flexible liquid-impermeable inner bag that is incorporated in the said trap portion and holds at least one end of the said absorptive body is further provided.

8. The absorptive product, according to claim 7, **characterized in that** the said inner bag has a folded portion that can evaginate.

9. The absorptive product, according to claim 8, **characterized in that** the said trap portion further has the openings to allow a part of the said inner bag's said folded portion to evaginate out of the said trap portion.

10. The absorptive product, according to any one of the claims 6 through 9, **characterized in that** the said individual-packaging sheet and the said trap sheet are made of the same sheet material.

11. The absorptive product, according to any one of the claims 1 through 5, **characterized in that** the other end of the said trap sheet has liquid-permeability.

12. The absorptive product, according to claims 11, **characterized in that** it further has a guiding section, joined to the portion of said trap sheet where it has liquid-permeability, facing the female wearer's urinary meatus to guide the urine to the absorptive body in an dispersed state.

13. The absorptive product, according to any one of the claims 1 through 12, **characterized in that** a pair of elastic members are further provided along the right and left side edges of the said individual-packaging sheet, joined in the stretched state to both the right and left side edges of the said individual-packaging sheet.

14. The absorptive product, according to any one of the claims 1 through 13, **characterized in that** the said absorptive body has a non-woven substrate in a sheet form and there are multiple liquid-absorbing portions coated on this non-woven substrate at prescribed intervals.

15. The absorptive product, according to claims 14, **characterized in that** the said absorptive body contains 60 weight % or more of SAP.

16. The absorptive product, according to any one of the claims 1 through 15, **characterized in that** the said absorptive body has a hydrodisintegrative characteristic.

17. The absorptive product, according to any one of the claims 1 through 16, **characterized in that** the said individual-packaging sheet makes up the absorptive product's liquid impermeable barrier material.

18. The method of producing the absorptive product, according to claim 9, **characterized in that** the method of producing the absorptive product involves a step to form a pair of cross-directional straight-line crena along both side edges of the single sheet material to make an opening on each side of this single sheet material, a step to fold the said single sheet material in the width direction in such a manner to include the said pair of crena in the folded portion, and a step to form the said trap portion by joining the folded portion that overlaps each other along the cross-sectional side edges of the said single sheet material.

19. The inner bag used for the absorptive product, according to claim 8, **characterized in that** it has a folded portion comprising the first pair of folded portion, one each on the right and left, formed by folding the right side and left side of the rectangular sheet toward the center, the second folded portion formed by folding the bottom end of the said sheet upward, the third folded portion formed by folding the top end of this second folded portion downward, and the hanger tape each of whose both ends is joined to the center of this third folded portion and the center of the top part of the said sheet.

20. The method of producing the inner bag, according to claim 19, **characterized in that** it has a step to form the first pair of folded portion, one each on the right and left, by folding the right side and left side of the rectangular sheet toward the center, a step to form the second folded portion by folding the bottom end of the said sheet upward, a step to form the third folded portion by folding the top end of this second folded portion downward, and a step to join both ends of the hanger tape each to the center of this third folded portion and the center of the top part of the said sheet.

21. The method of handling the absorptive product, according to any one of the claims 1 through 17, or the absorptive product produced by the method, according to any one of the claims 1 through 8, **characterized in that** it has a step to open the portion on the said other end of the said individual-packaging sheet by peeling off the said joining tape, a step to use the said absorptive product in this opened state, and a step, after use, to join, using the said joining tape, the said other end of the said individual-packaging sheet and one end of this individual-packaging sheet or the said trap sheet, by overlapping the said other end of the said individual-packaging sheet with the said trap sheet.

22. The method of handling the absorptive product, according to claim 21, **characterized in that** it further has a step to allow disposal of the used absorptive product while maintaining its joined state, by joining, after use, the other end of the said individual-packaging sheet and one end of this individual-packaging sheet or the said trap sheet.

23. The underpants, **characterized in that** they have a part to which the absorptive product, according to any one of the claims 1 through 17, or the absorptive product produced by the method, according to any one of the claims 1 through 8, is fit, in such a manner that it is detachable.

24. The underpants, **characterized in that** they have the absorptive product, according to any one of the claims 1 through 17, or the absorptive product produced by the method, according to any one of the claims 1 through 8, and a part to which this absorptive product is fit, in such a manner that it is detachable.

25. The underpants, according to claim 23 or 24, **characterized in that** the said part to fit is provided inside the underpants.

26. The underpants, according to any one of the claims 23 through 25, **characterized in that** the said joining tape of the absorptive product is further equipped with joining portions that enable detachable joining.

27. The underpants, according to claim 26, **characterized in that** the said joining portion is provided outside the underpants.

28. The underpants, according to any one of the claims 23 through 27, **characterized in that** the said underpants are of the plain knit product.
